# EUROPEAN PATENT SPECIFICATION

(11) **EP 4 051 088 B1**
(45) Date of publication and mention of the grant of the patent: **03.09.2025**
(21) Application number: 20797470.0
(22) Date of filing: 28.10.2020
(51) Int. Cl.: A61B 5/00, A61B 18/20, G16H 30/40, G16H 40/63, G16H 50/30, G16H 50/70, A61B 17/00, A61B 90/00

(54) **EVALUATING SKIN**
BEWERTUNG DER HAUT
ÉVALUATION DE LA PEAU

(30) Priority: 01.11.2019 EP 19206773
(43) Date of publication of application: 07.09.2022
(73) Proprietor: Koninklijke Philips N.V., 5656 AG Eindhoven (NL)
(72) Inventor: FERNANDO, Shakith, Devinda, 5656 AE Eindhoven (NL); VAN BREE, Karl, Catharina, 5656 AE Eindhoven (NL); THUMMA, Kiran, Kumar, 5656 AE Eindhoven (NL)
(74) Representative: Philips Intellectual Property & Standards
(86) International application number: PCT/EP2020/080220
(87) International publication number: WO 2021/083923

(56) References cited:
- WO-A1-2009/089292
- WO-A1-2018/160963
- US-A1- 2015 032 092
- US-A1- 2018 220 952
- US-A1- 2019 130 538

## Description

### FIELD OF THE INVENTION

This disclosure relates to evaluating skin and in particular to determining a skin type and/or a melanin index of skin.

### BACKGROUND OF THE INVENTION

Intense pulsed light (IPL) technology is a popular solution for many applications such as, but not limited to, photoepilation, lesion treatment, photo-rejuvenation, in-home personal care, professional personal care and medical settings. For photoepilation in a home environment, IPL photoepilation devices apply broad-spectrum light to the surface of the skin, targeting the melanin in the hair. Hair and the follicle (that are in their anagen phase of the growth cycle) absorbs this energy and goes into its resting phase. This prevents the regrowth of hair. For effective use of this IPL technology for hair removal (e.g. to minimise damage or irritation to the skin), the energy setting of the IPL photoepilation device should be adapted based on the skin tone of the skin. Some IPL photoepilation devices, e.g. the Philips Lumea Prestige, can detect the skin tone and select an appropriate energy setting. The skin tone can be detected and categorised into one of, e.g. six, different types. The skin types 1 to 6 can be broadly labelled as: 'white', 'beige', 'light brown', 'medium brown', 'dark brown and brownish black and darker'. Typically IPL photoepilation devices should not be used with darker skin as the skin will absorb energy in the light pulse rather the hair or follicle. In that case, if a skin tone of e.g. brownish black and darker, is detected, the device should not trigger a flash.

In addition to hair removal, light-based technologies can also be used for other types of dermatological treatments, including treating acne and skin lesions.

A current method of skin type detection in IPL devices uses reflectance spectroscopy, for example as described in "A portable reflectometer for a rapid quantification of cutaneous haemoglobin and melanin" by Feather J.W., Ellis D.J., and Leslie G., Phys. Med. Biol. 1988, 33, 711-722. In this technique, the ratio between two reflected wavelengths (red and near infrared - melanin has a higher absorption coefficient at these two wavelengths compared to water and haemoglobin) are used to compute a melanin index, which then used to compute skin type. These reflected energy signals are affected by temperature, ambient light, and the waveguide of the IPL device. Furthermore, the ratio can be calibrated against reference synthetic skin samples. Another alternative to this method is to use smartphone camera images of a skin region to compute skin tone. This is also very challenging due to specular reflectivity of skin, variance in illumination conditions, and variance in image sensor characteristics. Furthermore, the image measurement may require use of a colour calibration card.

Therefore improvements are desired to determining a skin type and/or a melanin index of skin, for example for use in setting an energy level for an IPL device.

Further examples of skin evaluation according to the state of the art are for instance described in US2018/0220952A1, US2019/0130538A1, WO2009/089292A1, US2015/0032092A1 and WO2018/160963A1.

### SUMMARY OF THE INVENTION

The invention is defined in the appended claims.

### SUMMARY OF THE DISCLOSURE

According to a first aspect, there is provided an apparatus for evaluating skin of a subject. The apparatus comprises a processing unit configured to receive one or more images of the skin of the subject from an imaging unit, with the imaging unit arranged to obtain images of the skin of the subject; process the one or more images to determine a skin type and/or a melanin index of the skin of the subject based on a density of a structure of a pigment network of the skin; and output a first signal indicating the determined skin type and/or melanin index. Thus the apparatus is able to determine a skin type and/or melanin index of the skin from image(s) of the skin.

In some embodiments, the processing unit is configured to determine a density of melanin in keratinocytes and/or melanocytes in the skin from the one or more images. In these embodiments, the processing unit is configured to determine the skin type and/or melanin index by comparing the determined density of melanin to respective thresholds corresponding to respective skin types and/or melanin indices.

In some embodiments, the processing unit is configured to determine a pattern of flanges and/or ridges of dermal papillae from the one or more images. In these embodiments, the processing unit is configured to determine the skin type and/or melanin index by comparing the determined pattern of flanges and/or ridges to respective patterns corresponding to respective skin types and/or melanin indices.

In some embodiments, the processing unit is configured to use a trained machine learning model to process the one or more images to determine the skin type and/or the melanin index of the skin of the subject based on the density of the structure of the pigment network. The trained machine learning model can be an artificial neural network, such as a deep neural network.

In some embodiments, the determined skin type comprises any of: normal skin, dry skin, oily skin, tanned skin, untanned skin, combination skin and a skin type on the Fitzpatrick scale.

In some embodiments, the determined melanin index comprises a number on a melanin scale.

In some embodiments, the apparatus further comprises a user interface configured to receive the first signal, and the first signal is configured to cause the user interface to output feedback to a user indicating the determined skin type and/or melanin index.

In some embodiments, the first signal is output to a control unit of a treatment device, and the control unit uses the first signal to determine one or more operational settings for the treatment device. Thus, these embodiments are useful where settings for a treatment operation by a treatment device are varied based on a skin type and/or melanin index, as the operational settings can be applied automatically by the treatment device in response to the first signal.

In some embodiments, the processing unit is configured to normalise the received one or more images for colour, and to process the one or more normalised images to determine the skin type and/or melanin index of the skin. In this way it can be ensured that the colour of the skin does not affect the determination of the skin type and/or melanin index, and instead the determination is based on the density of the structure of the pigment network.

According to a second aspect, there is provided a system that comprises an imaging unit arranged to obtain images of skin of a subject; and an apparatus according to the first aspect or any embodiment thereof.

In some embodiments, the system further comprises a treatment device that is for performing a treatment operation on the skin of the subject.

In some embodiments, the treatment device comprises a control unit that is configured to receive the first signal, and the control unit is configured to determine one or more operational settings for the treatment device based on the skin type and/or melanin index indicated in the first signal.

In these embodiments the treatment device can comprise the imaging unit and/or the apparatus. In alternative embodiments the treatment device can be separate from the apparatus.

In some embodiments, the system further comprises one or more light sources for illuminating the skin. These light source(s) can be used to illuminate the skin when the images are obtained.

In some embodiments, the light source(s) are configured to emit light having wavelengths in the visible spectrum, and emitted blue light has a higher intensity than light of other colours.

In some embodiments, the system further comprises a first polariser arranged to polarise the light emitted by the one or more light sources. In some embodiments, the system further comprises a second polariser that is arranged to polarise light incident from the skin onto the imaging unit. The use of polarised light and/or polarising the light incident on the imaging unit can improve the depth of the skin visible in the obtained images.

In embodiments that include a first polariser and a second polariser, the first polariser and the second polariser can be arranged orthogonally, or substantially orthogonally, with respect to each other.

According to a third aspect, there is provided a computer-implemented method for evaluating the skin of a subject, the method comprising: receiving one or more images of the skin of the subject from an imaging unit, with the imaging unit arranged to obtain images of the skin of the subject; processing the one or more images to determine a skin type and/or a melanin index of the skin of the subject based on a density of a structure of a pigment network of the skin; and outputting a first signal indicating the determined skin type and/or melanin index. Thus the method is able to determine a skin type and/or melanin index of the skin from image(s) of the skin.

In some embodiments, the step of processing comprises determining a density of melanin in keratinocytes and/or melanocytes in the skin from the one or more images. In these embodiments, the step of processing comprises determining the skin type and/or melanin index by comparing the determined density of melanin to respective thresholds corresponding to respective skin types and/or melanin indices.

In some embodiments, the step of processing comprises determining a pattern of flanges and/or ridges of dermal papillae from the one or more images. In these embodiments, the step of processing comprises determining the skin type and/or melanin index by comparing the determined pattern of flanges and/or ridges to respective patterns corresponding to respective skin types and/or melanin indices.

In some embodiments, the step of processing comprises using a trained machine learning model to process the one or more images to determine the skin type and/or the melanin index of the skin of the subject based on the density of the structure of the pigment network. The trained machine learning model can be an artificial neural network, such as a deep neural network.

In some embodiments, the determined skin type comprises any of: normal skin, dry skin, oily skin, tanned skin, untanned skin, combination skin and a skin type on the Fitzpatrick scale.

In some embodiments, the determined melanin index comprises a number on a melanin scale.

In some embodiments, the step of outputting comprises outputting the first signal to a user interface, and the method further comprises outputting, by the user interface, feedback to a user indicating the determined skin type and/or melanin index.

In some embodiments, the step of outputting comprises outputting the first signal to a control unit of a treatment device, and the method further comprises using, by the control unit, the first signal to determine one or more operational settings for the treatment device. Thus, these embodiments are useful where settings for a treatment operation by a treatment device are varied based on a skin type and/or melanin index, as the operational settings can be applied automatically by the treatment device in response to the first signal.

In some embodiments, the method further comprises the step of normalising the received one or more images for colour, and processing the one or more normalised images to determine the skin type and/or melanin index of the skin. In this way it can be ensured that the colour of the skin does not affect the determination of the skin type and/or melanin index, and instead the determination is based on the density of the structure of the pigment network.

In some embodiments, the method further comprises obtaining the one or more images using the imaging unit. In these embodiments, the method can further comprise illuminating the skin using one or more light sources when obtaining the one or more images.

In some embodiments, the step of illuminating comprises the light source(s) emitting light having wavelengths in the visible spectrum, and emitting blue light at a higher intensity than light of other colours.

In some embodiments, the method further comprises polarising light emitted by the one or more light sources. In some embodiments, the method further comprises polarising light incident from the skin onto the imaging unit. The use of polarised light and/or polarising the light incident on the imaging unit can improve the depth of the skin visible in the obtained images.

According to another aspect, there is provided a computer program product comprising a computer readable medium having computer readable code embodied therein, the computer readable code being configured such that, on execution by a suitable apparatus, computer or processing unit, the apparatus, computer or processing unit is caused to perform the method according to the third aspect, or any embodiment thereof.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is an illustration of an exemplary treatment device with which the invention can be used;
Fig. 2 is a block diagram of an exemplary system comprising an imaging unit and an apparatus according to various embodiments;
Fig. 3 is a plot illustrating an exemplary light spectrum for a light source that is to illuminate skin;
Fig. 4 shows six images of different types of skin;
Fig. 5 illustrates a pigment network in skin;
Fig. 6 is a flow chart illustrating an exemplary method for evaluating skin;
Fig. 7 shows four images of different types of skin;
Fig. 8 shows four images of different types of skin after normalisation for colour;
Fig. 9 is a plot showing an inferencing result for different skin types;
Fig. 10 is a flow chart illustrating a method of training a machine learning model for use in evaluating skin;
Fig. 11 is a plot showing an inferencing result for a training fold;
Fig. 12 is a plot showing an inferencing result for another training fold; and
Fig. 13 is a plot showing an inferencing result for another training fold.

### DETAILED DESCRIPTION OF THE EMBODIMENTS

As noted above, the techniques described herein can be used to evaluate skin shown in an image or images to determine a skin type and/or a melanin index of the skin. The techniques can be implemented by a treatment device that may make use of the determined skin type and/or melanin index (e.g. the techniques can be implemented by a processing unit in the treatment device), or implemented by a processing unit in a separate apparatus. As noted below, an exemplary type of treatment device that the skin type and/or melanin index can be used by is a treatment device that uses light pulses to remove hair and/or reduce hair growth. It will be appreciated that the determined skin type and/or melanin index do not have to be used by a treatment device, or for a purpose associated with a treatment device. For example the skin type and/or melanin index can be used to recommend a skin product (e.g. makeup or concealer), and/or assess a pigment disorder.

An imaging unit (e.g. a camera) is used to obtain one or more images of an area of skin on a subject. The imaging unit may be part of the treatment device (if applicable), part of a separate apparatus, or separate from both the treatment device and any apparatus that implements the techniques described herein. For example the imaging unit may be or include a camera on a device such as a smartphone.

It has been found that the skin type and/or melanin index is related to the structure of the skin, and this structure can be observed in image(s) of the skin. In particular, the density of a structure of a pigment network in the skin can be related to the skin type and/or melanin index. For darker skin types, the pigment network is typically a well-connected (i.e. densely-connected) and well-populated honeycomb structure, whereas for light skin types, the pigment network is disconnected and sparser (i.e. less dense), and in some cases the pigment is not perceivable. Thus according to the techniques described herein, one or more images of the skin are analysed to determine a skin type and/or a melanin index of the skin of the subject based on a density of a structure of a pigment network of the skin.

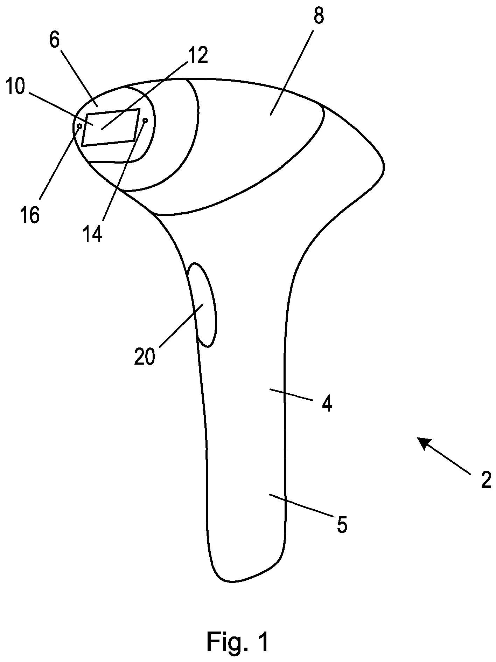

Fig. 1 is an illustration of an exemplary treatment device 2 that can be used to apply a light pulse to an area of skin. It will be appreciated that the treatment device 2 in Fig. 1 is merely presented as an example of a hand-held treatment device 2 that the invention can be used with, and the treatment device 2 is not limited to the form shown in Fig. 1 or to being a hand-held treatment device. As noted above, the invention is likewise not limited to being implemented in or with a treatment device 2.

The treatment device 2 in Fig. 1 is for use on a body of a subject (e.g. a person or an animal), and is to be held in one or both hands of a user during use. The treatment device 2 is to perform some treatment operation to hairs on the body of the subject using one or more light pulses when the treatment device 2 is in contact with a body part of the subject. The treatment operation can be removal of unwanted hairs by laser and/or light therapies (known as a photoepilation treatment or Intense Pulsed Light, IPL, treatment).

As described herein, the treatment device 2 is operated or used by a 'user', and the treatment device 2 is used on a body of a 'subject'. In some cases the user and the subject is the same person, i.e. the treatment device 2 is held in a hand and used by a user on themselves (e.g. used on the skin on their leg). In other cases the user and the subject are different people, e.g. the treatment device 2 is held in a hand and used by a user on someone else. In either case, it is difficult for a user to achieve complete coverage of the body region and/or avoid over-treating certain areas of the body region since there are little or no user-perceptible changes to the skin or hairs shortly after applying a light pulse.

The exemplary treatment device 2 comprises a housing 4 that includes at least a handle portion 5 and a head portion 6. The handle portion 5 is shaped to enable the user to hold the treatment device 2 with one hand. The head portion 6 is at a head end 8 of the housing 4, and the head portion 6 is to be placed into contact with the subject in order for the personal care operation to be performed on the body or skin of the subject at the position that the head portion 6 is in contact with the body or skin.

The treatment device 2 is for performing a treatment operation using light pulses. Thus, in Fig. 1 the head portion 6 comprises an aperture 10 that is arranged in or on the housing 4 so that the aperture 10 can be placed adjacent to or on (i.e. in contact with) the skin of the subject. The treatment device 2 includes one or more light sources 12 that are for generating light pulses that are to be applied to the skin of the subject via the aperture 10 and effect a treatment operation. The one or more light sources 12 are arranged in the housing 4 so that the light pulses are provided from the one or more light sources 12 through the aperture 10. The aperture 10 may be in the form of an opening at the head end 8 of the housing 4, or it may be in the form of a window (including a waveguide) that is transparent or semi-transparent to the light pulses (i.e. the light pulses can pass through the window).

In the exemplary embodiment shown in Fig. 1, the aperture 10 has a generally rectangular shape, which results in a generally rectangular-shaped skin treatment region on the skin. It will be appreciated that the aperture 10 can have any other desired shape. For example the aperture 10 can be square, elliptical, circular, or any other polygonal shape.

The one or more light sources 12 can generate light pulses of any suitable or desired wavelength (or range of wavelengths) and/or intensities. For example, the light source 12 can generate visible light, infra-red (IR) light and/or ultraviolet (UV) light. Each light source 12 can comprise any suitable type of light source, such as one or more light emitting diodes (LEDs), a (Xenon) flash lamp, a laser or lasers, etc. The light source(s) 12 can provide light pulses with spectral content in the 560-1200 nanometre (nm) range for a duration of around 2.5 milliseconds (ms), as these wavelengths heat melanin in the hair and hair root by absorption, which puts the hair follicles in a resting phase, preventing hair regrowth.

The one or more light sources 12 are configured to provide pulses of light. That is, the light source(s) 12 are configured to generate light at a high intensity for a short duration (e.g. less than 1 second). The intensity of the light pulse should be high enough to effect the treatment operation on the skin or body part adjacent the aperture 10.

The illustrated treatment device 2 also includes two skin contact sensors 14, 16 positioned on or in the head portion 6 that are used to determine whether the head portion 6 is in contact with the skin. The skin contact sensors 14, 16 measure a parameter that is indicative of whether the head portion 6 is in contact with skin, and generate respective measurement signals that comprise a time-series of measurements of the parameter. The measurement signals can be processed to determine if the head portion 6 is in contact with skin. Typically a skin contact sensor is used in a treatment device 2, particularly a photoepilator, to make sure that the treatment device 2 is correctly in contact with skin before a light pulse is generated to avoid the light pulse being directed into the eyes of the user or subject.

In some embodiments the parameter can be capacitance, and so the skin contact sensors 14, 16 can measure capacitance via a respective pair of electrical contacts or electrodes on the surface of the head portion 6, with the measured capacitance being indicative of whether there is skin contact. In alternative embodiments, the parameter can be an intensity or level of light, and so the skin contact sensors 14, 16 can be light sensors that measure an intensity or level of light incident on the light sensor, with the measured intensity or level being indicative of whether there is skin contact (e.g. less/no light could indicate skin contact as the skin obscures the light sensors 14, 16, and vice versa). In other alternative embodiments, the parameter can be a measure of contact pressure, and so the skin contact sensors 14, 16 can measure contact pressure via respective pressure sensors or mechanical switches, with the measured contact pressure being indicative of whether there is skin contact.

The illustrated treatment device 2 also includes a user control 20 that can be operated by the user to activate the treatment device 2 so that the head portion 6 performs the required treatment operation on the body of the subject (e.g. the generation of one or more light pulses by the one or more light source(s) 12). The user control 20 may be in the form of a switch, a button, a touch pad, etc.

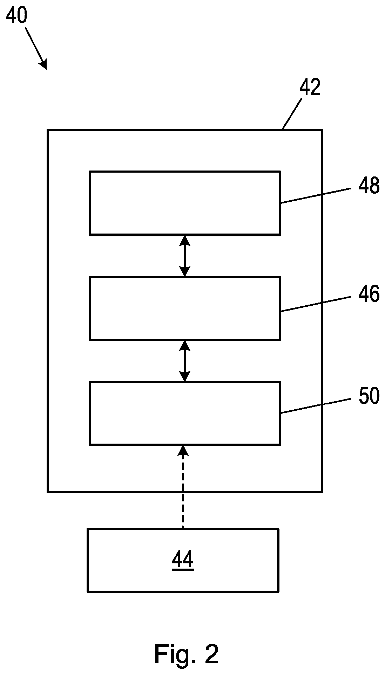

Fig. 2 is a block diagram of an exemplary system 40 comprising an apparatus 42 for evaluating skin of a subject and an imaging unit 44. In some implementations the treatment device 2 can be considered part of the system 40, although the treatment device 2 is not shown in Fig. 2. As noted above, the apparatus 42 can be a separate device to the treatment device 2, and thus the apparatus 42 may be in the form of an electronic device, such as a smart phone, smart watch, tablet, personal digital assistant (PDA), laptop, desktop computer, remote server, smart mirror, etc. In other embodiments, the apparatus 42, and particularly the functionality according to the invention provided by the apparatus 42, is part of the treatment device 2.

The imaging unit 44 is provided to generate one or more images (or a video sequence) of one or more areas of skin of the subject. The imaging unit 44 may include any suitable components for capturing an image, for example a charge-coupled device (CCD) and one or more lenses and/or mirrors. In some embodiments, the imaging unit 44 is a camera, such as a digital camera. In some embodiments, the imaging unit 44 is a camera that is set up to provide microscopic images of the skin (i.e. the imaging unit 44 provides enlarged or zoomed in images of the skin). In some embodiments, one or more additional optical components are associated with, or part of, the imaging unit 44. For example a polariser can be provided that is placed in front of the imaging unit 44 in order to polarise light that is incident on the imaging unit 44. The use of a polariser can improve the depth of the skin area visible in the image (i.e. a polariser enables light to be observed at different penetration depths in the skin). In some embodiments, one or more light source(s) can be provided for illuminating the skin area to be imaged, while the image is obtained. These light source(s) are separate from any light source(s) 12 in the treatment device 2 that are used to effect a treatment operation. In these embodiments, a polariser can be provided to polarise the light emitted by the light source(s) before it illuminates the skin. The light source(s) for illuminating the skin for the purpose of obtaining the image(s) can be any suitable light source, e.g. one or more LEDs.

In particular embodiments, a first polariser can be provided that polarises the light emitted by one or more light source(s) before the light illuminates the skin, and a second polariser can be provided that polarises light that is incident on the imaging unit 44 from the skin. The first polariser and the second polariser can be arranged so that they are 'crossed', i.e. they are arranged so that their polarisation directions are orthogonal (i.e. at 90° with respect to each other), or substantially orthogonal (i.e. around 90° with respect to each other), to each other. The use of crossed polarisers can improve the depth of the skin area visible in the image, and thus enables the structure of the skin to be observed.

The imaging unit 44 is shown in Fig. 2 as being separate from the apparatus 42, but it will be appreciated that in other embodiments the imaging unit 44 can be integral with or part of the apparatus 42. In embodiments where the imaging unit 44 is separate from the apparatus 42, the imaging unit 44 may be part of the treatment device 2, or it may also be separate from the treatment device 2. In embodiments where the imaging unit 44 is part of the treatment device 2, the imaging unit 44 can be arranged in the treatment device 2 close to the aperture 10 so that images can be obtained when the treatment device 2 is on or close to the skin.

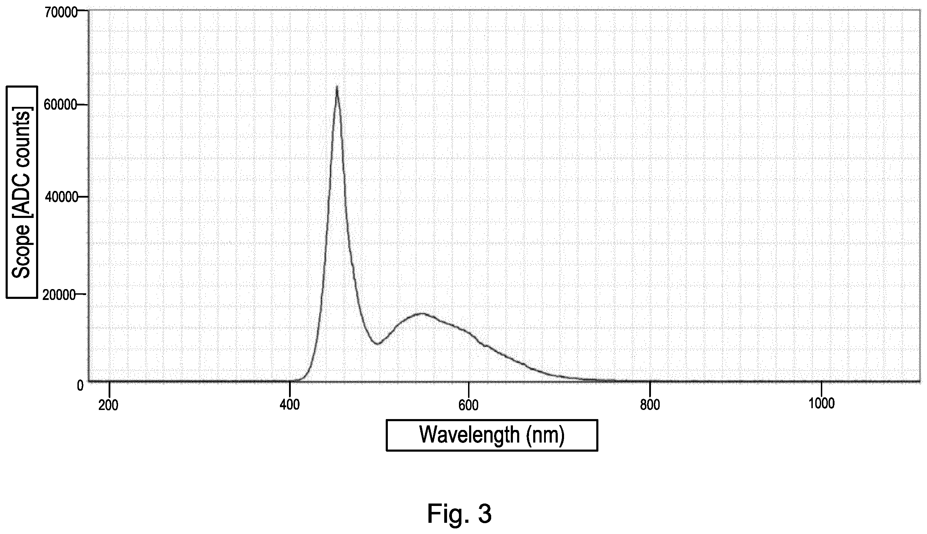

In exemplary embodiments where one or more light source(s) are provided for illuminating the skin area to be imaged, the one or more light source(s) can emit light at a particular wavelength(s), or in particular ranges of wavelengths. The selected light spectrum can enable structures of the skin to be identified and/or analysed in the resulting image(s), such as a pigment network. Fig. 3 is a plot illustrating an exemplary light illumination spectrum for light source(s) that are to illuminate the skin when obtaining image(s). Thus it can be seen that the light source(s) can emit light having wavelengths in the range 400 nanometres (nm) to 760nm (i.e. visible light), with the highest intensity of light emitted being around 450 nm (i.e. blue light). Light in the range 500 nm - 700 nm (i.e. green - red light) is also emitted, but at a (much) lower intensity than the blue light. In the example of Fig. 3, the intensity of the emitted light reduces significantly towards the red end of the spectrum. In conjunction with the crossed polarisers described above, light source(s) having the light illumination spectrum in Fig. 3 (or similar) enables images to be obtained from the depth of the skin where the melanin/pigment network is located. These images will not include specular components, or red components from deeper skin areas caused by the presence of blood vessels.

The apparatus 42 comprises a processing unit 46 that generally controls the operation of the apparatus 42 and enables the apparatus 42 to perform the method and techniques described herein. Briefly, the processing unit 46 receives one or more images from the imaging unit 44, processes the image(s) to determine a skin type and/or a melanin index of the skin of the subject based on a density of a structure of a pigment network of the skin as shown in those images, and outputs a first signal indicating the determined skin type and/or melanin index.

Thus the processing unit 46 can be configured to receive the image(s) from the imaging unit 44, either directly in embodiments where the imaging unit 44 is part of the apparatus 42, or via another component in embodiments where the imaging unit 44 is separate from the apparatus 42. In either case, the processing unit 46 can include or comprise one or more input ports or wires for receiving the images (or signals carrying information representing the image(s)) from the imaging unit 44 or the other component as appropriate. The processing unit 46 can also include or comprise one or more output ports or wires for outputting the signal indicating whether hairs on the area of skin have been treated with a light pulse.

The processing unit 46 can be implemented in numerous ways, with software and/or hardware, to perform the various functions described herein. The processing unit 46 may comprise one or more microprocessors or digital signal processors (DSPs) that may be programmed using software or computer program code to perform the required functions and/or to control components of the processing unit 46 to effect the required functions. The processing unit 46 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 46 can comprise or be associated with a memory unit 48. The memory unit 48 can store data, information and/or signals (including image(s)) for use by the processing unit 46 in controlling the operation of the apparatus 42 and/or in executing or performing the methods described herein. In some implementations the memory unit 48 stores computer-readable code that can be executed by the processing unit 46 so that the processing unit 46 performs one or more functions, including the methods described herein. In particular embodiments, the program code can be in the form of an application for a smart phone, tablet, laptop, computer or server. The memory unit 48 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In the embodiment shown in Fig. 2, as the apparatus 42 is shown as being separate from the imaging unit 44, the apparatus 42 also includes interface circuitry 50 to enable the apparatus 42 to receive the image(s) from the imaging unit 44. The interface circuitry 50 in the apparatus 42 enables a data connection to and/or data exchange with other devices, including any one or more of the imaging unit 44, the treatment device 2, servers, databases, user devices, and sensors. The connection to the imaging unit 44 (or any electronic device, such as treatment device 2) may be direct or indirect (e.g. via the Internet), and thus the interface circuitry 50 can enable a connection between the apparatus 42 and a network, or directly between the apparatus 42 and another device (such as imaging unit 44 and/or treatment device 2), via any desirable wired or wireless communication protocol. For example, the interface circuitry 50 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 50 (and thus apparatus 42) may include one or more suitable antennas for transmitting/receiving over a transmission medium (e.g. the air). Alternatively, in the case of a wireless connection, the interface circuitry 50 may include means (e.g. a connector or plug) to enable the interface circuitry 50 to be connected to one or more suitable antennas external to the apparatus 42 for transmitting/receiving over a transmission medium (e.g. the air). The interface circuitry 50 is connected to the processing unit 46.

Although not shown in Fig. 2, the apparatus 42 may comprise one or more user interface components that includes one or more components that enables a user of apparatus 42 to input information, data and/or commands into the apparatus 42, and/or enables the apparatus 42 to output information or data to the user of the apparatus 42. The user interface can comprise any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

It will be appreciated that a practical implementation of an apparatus 42 may include additional components to those shown in Fig. 2. For example the apparatus 42 may also include a power supply, such as a battery, or components for enabling the apparatus 42 to be connected to a mains power supply.

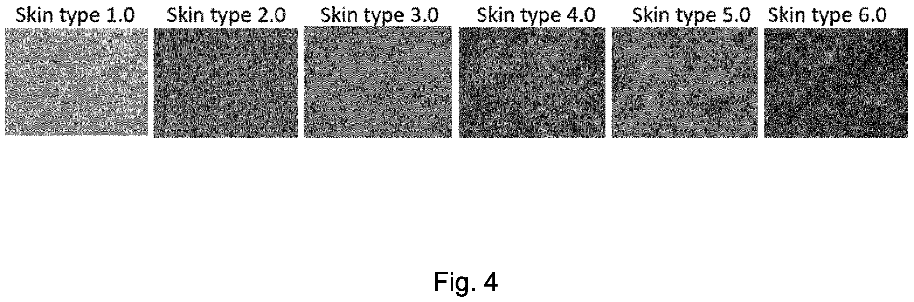

Fig. 4 shows six exemplary images of different types of skin. These images were obtained using an imaging unit 44 with the skin illuminated with light according to the spectrum shown in Fig. 3, and with crossed-polarisers. Each of the images shows one of the six different skin types outlined above (i.e. types 1 to 6: 'white', 'beige', 'light brown', 'medium brown', 'dark brown and brownish black and darker' respectively).

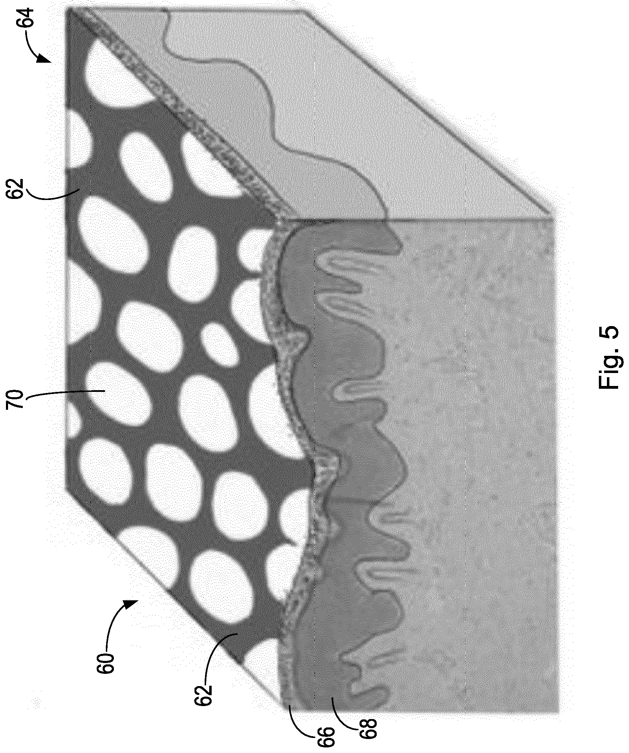

A general description of the structure of skin is found in "Standardization of terminology in dermoscopy/dermatoscopy: Results of the third consensus conference of the International Society of Dermoscopy" by Kittler et al., and an illustration of a normal pigment network is shown in Fig. 5. Fig. 5 can be found at
https://dermoscopedia.org/Pigment_network.

The pigment network 60 consists of a grid of intersecting pigmented "lines" 62 forming a honeycomb pattern 64. The anatomic basis of the pigment network 60 is melanin in keratinocytes and/or in melanocytes along the dermal-epidermal junction (epidermis 66 and dermis 68), representing the way the rete ridge pattern of the epidermis 66 appears when viewed in the horizontal plane. The less-pigmented "holes" 70 of the pigment network 60 correspond to tips of the dermal papillae and the overlying suprapapillary plates of the epidermis 66. A wide diameter of dermal papillae would correspond dermoscopically to wider network "holes" 70, whereas narrow dermal papillae would result in a denser "sieve" of the grid. The pigment network 60 may not be visible if the rete ridge pattern contains less melanin pigment. The patterns shown in the six microscopic images in Fig. 4 correspond to the melanin distribution along rete ridges along the top down view.

Thus, a density of a structure of a pigment network in the skin can be observed or determined from image(s) of the skin, and the density is related to the skin type and/or melanin index. For darker skin types (e.g. skin types 5.0 or 6.0), the pigment network typically has a well-connected (e.g. densely-connected) and well-populated honeycomb structure, whereas for light skin types (e.g. skin types 1.0 and 2.0), the structure of the pigment network is disconnected and sparser, and in some cases the pigment is not perceivable. Skin structure is also described generally in "Advances in Dermoscopy of Pigmented Lesions" by P. Kumarasinghe, Pigmentary Skin Disorders, pp 79-92.

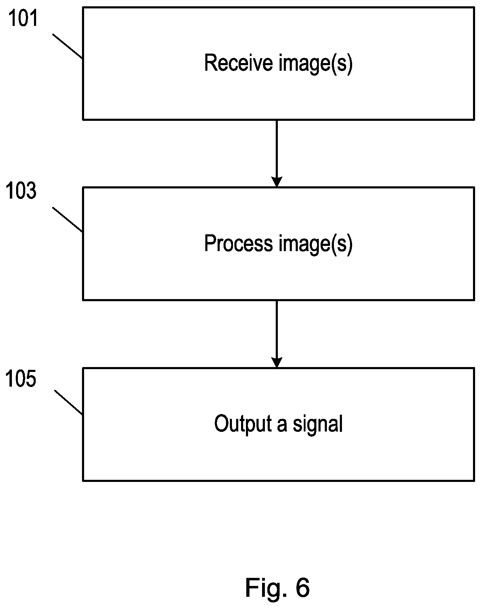

The flow chart in Fig. 6 illustrates an exemplary method according to the techniques described herein for evaluating skin of a subject. One or more of the steps of the method can be performed by the processing unit 46 in the apparatus 42, in conjunction with either of the memory unit 48 and interface circuitry 50 of the apparatus 42, and/or the imaging unit 44, as appropriate. The processing unit 46 may perform the one or more steps in response to executing computer program code that can be stored on a computer readable medium, such as, for example, the memory unit 48.

In step 101, one or more images of an area of skin are received. The image(s) can be received directly from imaging unit 44, for example in real-time or near real-time as the images are generated by the imaging unit 44. Alternatively, the image(s) may have been previously generated by the imaging unit 44 and stored for subsequent analysis, for example in the memory unit 48, in a memory unit associated with the treatment device 2 or imaging unit 44, or in a remote database, in which case step 101 can comprise the processing unit 46 obtaining or retrieving the image(s) from the storage location (e.g. from memory unit 48, etc.). The image(s) received in step 101 relate to a particular subject. Where multiple images are received, they may include multiple images of the same area of skin. The multiple images may also or alternatively include images of skin on different parts of the body of the subject.

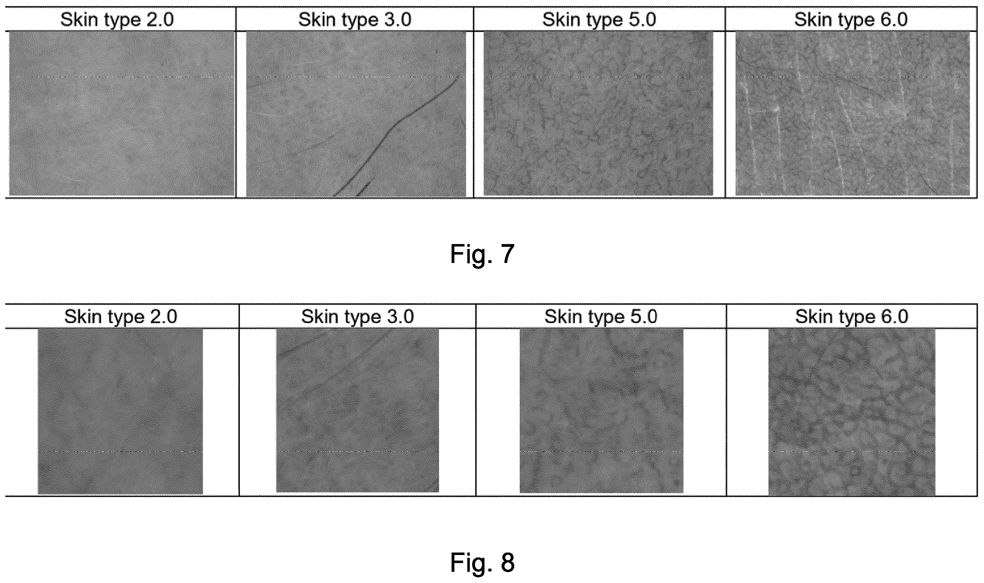

Fig. 7 shows images of four different types of skin that can be received or obtained in step 101. It will be appreciated that such different images would not be obtained from a single subject in a single iteration of step 101, and instead Fig. 7 merely illustrates the different types of skin that could be shown in images that can be obtained in step 101. Although the four images in Fig. 7 are labelled according to a floating-point continuous classification (similar to a discrete Fitzpatrick skin type), it will be appreciated that this classification is not known at this stage of the method.

In step 103, the one or more images are processed to determine a skin type and/or a melanin index of the skin in the image(s) based on a density of a structure of a pigment network of the skin.

Step 103 can comprise determining the skin type as any of normal skin, dry skin, oily skin, tanned skin, untanned skin, combination skin (i.e. a combination of the preceding types) and a skin type on the Fitzpatrick scale. In addition or alternatively, step 103 can comprise determining the melanin index as a score on a melanin scale (e.g. a score in the range 0-1000, or similar).

In some embodiments, step 103 can comprise determining the skin type and/or melanin index based on the density of the structure of the pigment network of the skin relating to the presence of melanin in keratinocytes and/or melanocytes in the structure. In some embodiments the skin type and/or melanin index can be based on the presence of melanin in keratinocytes and/or melanocytes along a dermal-epidermal junction in the skin. In particular, step 103 can comprise determining a density of melanin in keratinocytes and/or melanocytes in the skin. In some embodiments, the skin type and/or melanin index can be determined by comparing the determined density of melanin to respective thresholds corresponding to respective skin types and/or melanin indices.

In addition or alternatively, step 103 can comprise determining the skin type and/or melanin index based on the density of the structure of the pigment network of the skin relating to the presence of flanges and/or ridges of dermal papillae in the skin. In particular, step 103 can comprise determining a pattern of flanges and/or ridges of dermal papillae from the one or more images, and determining the skin type and/or melanin index by comparing the determined pattern of flanges and/or ridges to respective patterns corresponding to respective skin types and/or melanin indices.

In some preferred embodiments of step 103, a trained machine learning model (MLM) is used to process the one or more images to determine the skin type and/or the melanin index of the skin. The MLM is trained to determine the skin type and/or melanin index of the skin based on the density of the structure of the pigment network. The MLM can be any suitable type of MLM, for example a classical machine learning model such as feature extraction with support vector machines, decision trees, random forests, etc., or an artificial neural network, such as a deep neural network, that has multiple layers between input and output layers and which identifies a linear or non-linear relationship between the input and output layers. The MLM makes an evaluation for each image or set of images of a skin area to determine a skin type and/or melanin index. In some embodiments the MLM directly receives the image(s) and performs all required analysis and processing of the images (e.g. determines the density of the structure of the pigment network) in order to determine a skin type and/or melanin index. This is particularly the case for a MLM that is an artificial neural network, such as a deep neural network. In other embodiments, for example in the case of the use of a classical MLM, the image(s) can be processed before being provided to the MLM, for example to determine values for the density of the structure of the pigment network, and these values can be provided to the MLM for analysis (optionally in addition to the image(s)) to determine the skin type and/or melanin index. In some embodiments, separate trained MLMs (of the same type or different types) can be provided to determine the skin type and the melanin index respectively.

In some embodiments, prior to step 103, or as an initial operation in step 103, the received one or more images can be normalised for colour (i.e. the colour information is removed from the image(s), leaving a greyscale image), and the colour-normalised image(s) can be processed to determine the skin type and/or melanin index of the skin. Normalising the image(s) for colour removes colour from the decision process, which means that the decision on skin type and/or melanin index can be based on the density of the structure of the pigment network. Fig. 8 shows images of four different types of skin that have been normalised for colour. It should be noted that these images are of different areas of skin of different subjects to the images in Fig. 7. It will be appreciated that such different images would not be obtained from a single subject in a single iteration of step 101, and instead Fig. 8 merely illustrates the types of skin that could be shown in colour-normalised images. As with Fig. 7, although the four images in Fig. 8 are labelled according to a floating-point continuous classification (similar to a discrete Fitzpatrick skin type), it will be appreciated that this classification will not be known until step 103 is complete.

After a skin type and/or melanin index has been determined in step 103, in step 105 a signal is output indicating the determined skin type and/or melanin index. The signal may be provided to a user interface component of the apparatus 42 or treatment device 2 and the signal is configured to cause the user interface component to indicate the determined skin type and/or the determined melanin index. The user of the treatment device 2 could use the indication to determine whether to trigger a light pulse at the current position of the treatment device 2, or whether to adjust a power setting of one or more light source(s) 12 in the treatment device 2 according to the skin type. As another example, where the apparatus 42 is in the form of a smartphone or similar type of device, the feedback on the determined skin type and/or determined melanin index can be provided to the user or subject via an app (software application) executing on the apparatus 42. Those skilled in the art will be aware of other ways in which feedback on the determined skin type and/or determined melanin index can be provided to a user, e.g. including using a display screen, a loudspeaker, haptic feedback, etc.

Alternatively (or in addition), where the treatment device 2 can automatically trigger a light pulse or other treatment operation if the conditions are suitable (e.g. the treatment device 2 is in contact with skin, the light source(s) 12 are charged ready to generate a light pulse, etc.), the signal can be provided to a control unit of the treatment device 2, and the control unit can use the first signal to determine one or more operational settings for the treatment device 2. For example, the control unit can use the indication of the determined skin type and/or melanin index in the first signal to take the decision on whether, and/or the energy/power to use, to treat the area of skin currently adjacent to the aperture 10 with a light pulse.

In alternative embodiments, the signal indicating the determined skin type and/or melanin index can be used to provide recommendations or suggestions for particular skin care products and/or makeup products the subject could use. For example the determined skin type could be used to identify skin care products that are suitable for that skin type (or vice versa). As another example the determined melanin index could be used to recommend a particular concealer.

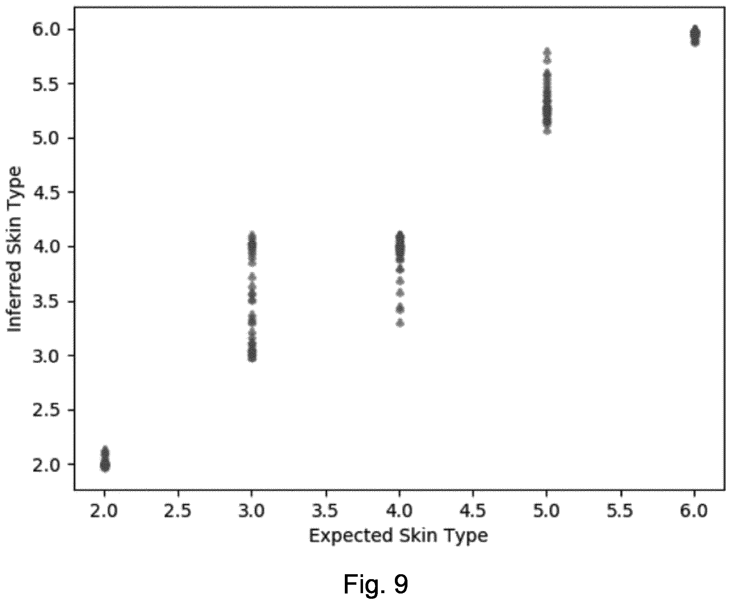

The plot in Fig. 9 shows the performance of a deep neural network in identifying skin type from a number of different colour-normalised images for different types of skin based on the density of the structure of the pigment network of the skin. Fig. 9 plots the actual skin type ('Expected Skin Type') against the skin type identified by the deep neural network ('Inferred Skin Type'). Thus, it can be seen that the neural network was able to accurately able to identify the skin type from the image(s), with accuracy being particularly high for the skin types at either end of the range (i.e. skin types 2.0 and 6.0).

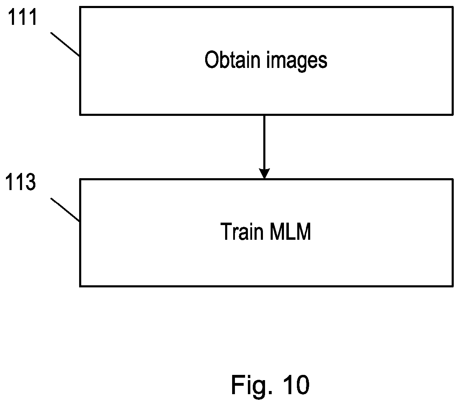

The MLM used in embodiments of step 103 will be trained prior to use. The flow chart in Fig. 10 illustrates a method of training a MLM for use in the apparatus for evaluating skin according to the invention, to determine a skin type and/or melanin index. The training method in Fig. 10 may be performed by any suitable apparatus or device, including apparatus 42, although it will be appreciated that the method in Fig. 10 does not need to be performed by the same apparatus or device that performs Fig. 6. For example the training method in Fig. 10 can be performed by a server or computer in a central location, and the trained MLM (or computer code representing the trained MLM) distributed to various apparatus 42 for use in evaluating skin according to the method in Fig. 6.

In embodiments where the apparatus 42 implements the method in Fig. 6, one or more of the steps of the method can be performed by the processing unit 46, in conjunction with either of the memory unit 48 and interface circuitry 50 of the apparatus 42, and/or the imaging unit 44, as appropriate. The processing unit 46 may perform the one or more steps in response to executing computer program code that can be stored on a computer readable medium, such as, for example, the memory unit 48.

In order to train the MLM, a training data set is required. The training data set comprises a plurality of images of skin for one or more test subjects (and which may or may not include the subject that the method of Fig. 6 is used on). The plurality of images may be of skin on different parts of the body of the one or more test subjects. Each image in the training data set is annotated with an indication of the skin type and/or melanin index (depending on which parameter the MLM is to be trained to identify). This annotation may have been made manually by a user or other person. The training data set should include at least one image (but preferably a plurality of images) relating to each of the different skin types and/or melanin indices that are to be identified by the trained MLM. It will be appreciated that the larger the training data set, the more accurate the resulting MLM is likely to be.

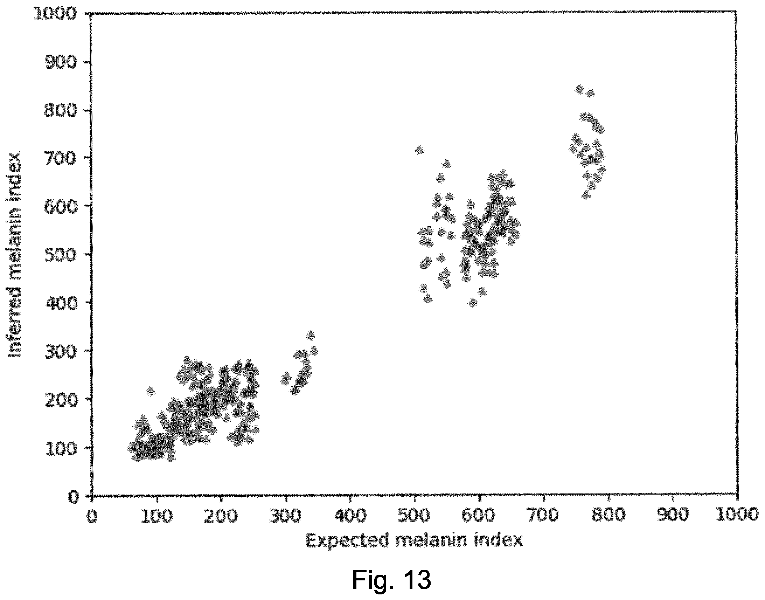

The training data set is obtained in step 111 of Fig. 13. This step can comprise collecting the images and associated annotations (e.g. using an imaging unit and user interface), or retrieving the images for the training data set from a database or other electronic storage device.

Next, in step 113, an MLM is trained using the plurality of images in the training data set so that the MLM is able to distinguish skin types and/or melanin indices (depending on which parameter(s) the MLM is to be able to identify). The MLM is trained to distinguish between the images based on the density of the structure of the pigment network visible in the images. As noted above for darker skin types (e.g. skin types 5.0 or 6.0), the pigment network typically has a well-connected and well-populated honeycomb structure, whereas for light skin types (e.g. skin types 1.0 and 2.0), the pigment network is disconnected and sparser, and in some cases the pigment is not perceivable. Thus, the MLM can be trained to differentiate skin types and/or melanin index based on the density of the pigment network.

In particular the MLM can be trained to determine skin type and/or melanin index based a density relating to the presence of melanin in keratinocytes and/or melanocytes in the structure of the pigment network and/or the presence of melanin in keratinocytes and/or melanocytes along a dermal-epidermal junction in the skin, for example by determining a density of melanin in keratinocytes and/or melanocytes in the skin. In addition or alternatively, the MLM can be trained to determine skin type and/or melanin index based a density relating to the presence of flanges and/or ridges of dermal papillae in the skin, for example a pattern of flanges and/or ridges of dermal papillae. Techniques for training MLMs using a training data set are known to those skilled in the art, and details are not provided herein. Nevertheless, as one example, the MLM can be trained using cross-validation where the MLM is trained using a subset of the images in the training data set and the trained MLM is tested for accuracy using the one or more of the other images in the training data set. This training and testing can be repeated for different subsets of the images in the training data set in order to arrive at the final trained MLM. The trained MLM can then be used in step 103 of Fig. 6.

In some embodiments the images in the training data set can be normalised for colour before they are used to train the MLM. In this way, during the training of the MLM the MLM is then biased towards skin structures in the images rather than colour differences.

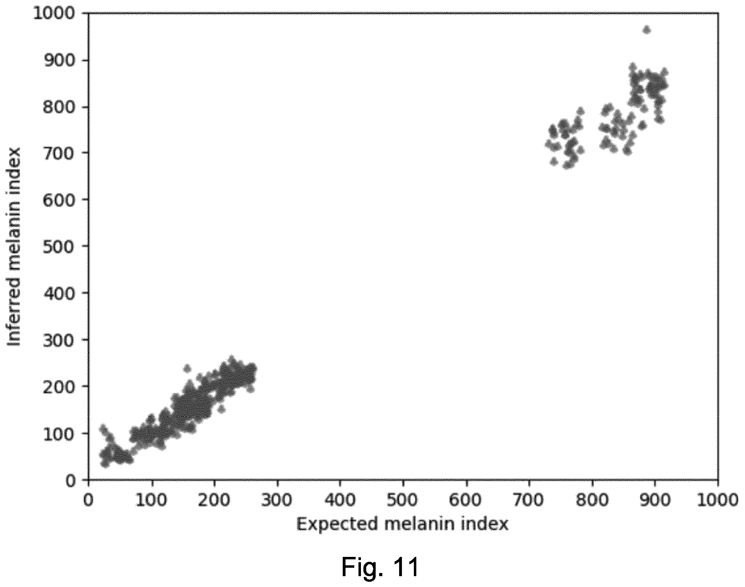

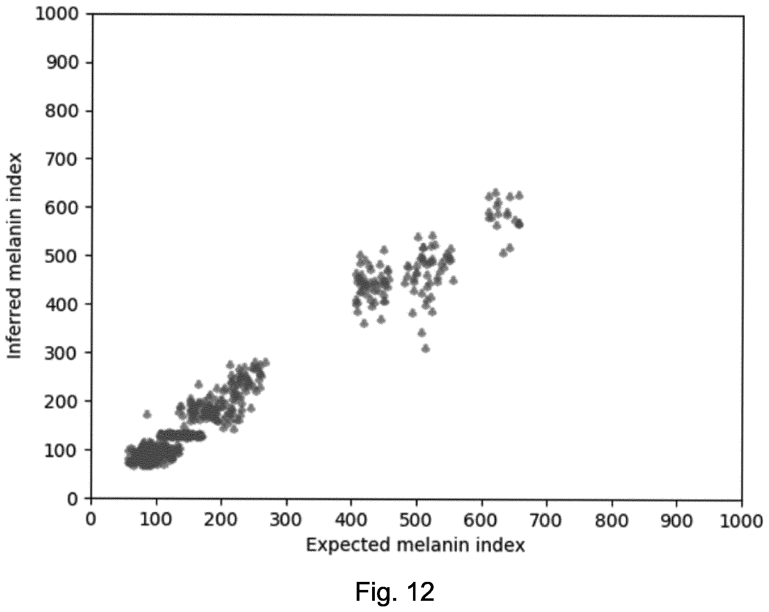

In an exemplary implementation, skin data (images) of 27 subjects was collected spanning all different skin types, with at least 5 subjects per skin type. These images included images of skin both before and after seasonal tanning, and also images of skin from several different body locations: cheek, inner arm, outer arm and leg. A reference melanin index was also measured for each skin patch shown in each image. A MLM (e.g. a deep neural network) was trained with the above-collected data using typical training techniques. To prove generalisability of skin structure for melanin index (and skin type) quantification, the dataset (the images of the 27 subjects) was split into 'folds' for a cross-fold validation experiment. Each fold consists of a training set (of 24 subjects from 27 subjects) and a validation set (of 3 subjects from 27 subjects). The training set and validation set are mutually exclusive. Each fold consists of a different permutation of subjects spilt into a training set and a validation set. For each fold, the training set was used to train the MLM, and the validation set was used to test and evaluate the trained MLM. Therefore, the MLM is tested and evaluated with images of skin structures from 3 subjects that were not included in the training set, and which the MLM has not seen before. The plots in Figs. 11, 12 and 13 show inferencing results obtained by the trained MLM according to three different folds of the original dataset. Thus each plot shows the performance of a deep neural network in identifying melanin index for a validation set of images for a particular fold. Each figure plots the actual melanin index ('Expected melanin index') against the melanin index identified by the deep neural network ('Inferred melanin index'). Thus, it can be seen from each figure that the neural network was able to accurately able to identify the melanin index with a minimal error spread.

There is therefore provided improvements in determining a skin type and/or a melanin index of skin. These improvements can, for example, be for use in setting an energy level for an IPL device or in adjusting the energy level of an IPL treatment for every location to be flashed, which can improve a comfort to efficacy ratio for the IPL user.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (42) for evaluating the skin of a subject, the apparatus (42) comprising a processing unit (46) configured to:
receive one or more images of the skin of the subject from an imaging unit (44), wherein the imaging unit (44) is arranged to obtain images of the skin of the subject;
process the one or more images to determine a skin type and/or a melanin index of the skin of the subject; and
output a first signal indicating the determined skin type and/or melanin index;
**characterised in that** the determination of the skin type and/or the melanin index of the skin is based on a density of a structure of a pigment network of the skin.

2. An apparatus (42) as claimed in claim 1, wherein the processing unit (46) is further configured to determine a density of melanin in keratinocytes and/or melanocytes in the skin from the one or more images.

3. An apparatus (42) as claimed in any of claims 1 or 2, wherein the processing unit (46) is further configured to determine a pattern of flanges and/or ridges of dermal papillae from the one or more images.

4. An apparatus (42) as claimed in any of claims 1-3, wherein the processing unit (46) is configured to use a trained machine learning model to process the one or more images to determine the skin type and/or the melanin index of the skin of the subject based on the density of the structure of the pigment network.

5. An apparatus (42) as claimed in any of claims 1-4, wherein the determined skin type comprises any of: normal skin, dry skin, oily skin, tanned skin, untanned skin, combination skin and a skin type on the Fitzpatrick scale.

6. An apparatus (42) as claimed in any of claims 1-5, wherein the apparatus (42) further comprises a user interface configured to receive the first signal, and wherein the first signal is configured to cause the user interface to output feedback to a user indicating the determined skin type and/or melanin index.

7. An apparatus (42) as claimed in any of claims 1-6, wherein the first signal is output to a control unit of a treatment device (2), and wherein said control unit uses the first signal to determine one or more operational settings for the treatment device (2).

8. An apparatus (42) as claimed in any of claims 1-7, wherein the processing unit (46) is configured to normalise the received one or more images for colour, and to process the one or more normalised images to determine the skin type and/or melanin index of the skin.

9. A system, comprising:
an imaging unit (44) arranged to obtain images of skin of a subject; and
an apparatus (42) as claimed in any of claims 1-8.

10. A system as claimed in claim 9, further comprising:
one or more light sources (12) for illuminating the skin.

11. A system as claimed in claim 10, wherein the one or more light sources (12) are configured to emit light having wavelengths in the visible spectrum, and wherein emitted blue light has a higher intensity than light of other colours.

12. A computer-implemented method for evaluating the skin of a subject, the method comprising:
receiving (101) one or more images of the skin of the subject from an imaging unit, with the imaging unit arranged to obtain images of the skin of the subject;
processing (103) the one or more images to determine a skin type and/or a melanin index of the skin of the subject;
outputting (105) a first signal indicating the determined skin type and/or melanin index;
**characterised in that** the determination of the skin type and/or the melanin index of the skin is based on a density of a structure of a pigment network of the skin.

13. A computer program product comprising a computer readable medium having computer readable code embodied therein, the computer readable code being configured such that, on execution by a suitable computer or processing unit, the computer or processing unit is caused to perform the method of claim 12.

## Patentansprüche

1. Einrichtung (42) zum Bewerten der Haut eines Subjekts, wobei die Einrichtung (42) eine Verarbeitungseinheit (46) umfasst, die dazu konfiguriert ist:
ein oder mehrere Bilder der Haut des Subjekts von einer Bildgebungseinheit (44) zu empfangen, wobei die Bildgebungseinheit (44) dazu eingerichtet ist, Bilder der Haut des Subjekts zu erhalten;
das eine oder die mehreren Bilder zu verarbeiten, um einen Hauttyp und/oder einen Melaninindex der Haut des Subjekts zu bestimmen; und
ein erstes Signal auszugeben, das den bestimmten Hauttyp und/oder Melaninindex angibt;
**dadurch gekennzeichnet, dass** die Bestimmung des Hauttyps und/oder des Melaninindex der Haut auf einer Dichte einer Struktur eines Pigmentnetzes der Haut basiert.

2. Einrichtung (42) nach Anspruch 1, wobei die Verarbeitungseinheit (46) weiter dazu konfiguriert ist, eine Dichte von Melanin in Keratinozyten und/oder Melanozyten in der Haut aus dem einen oder den mehreren Bildern zu bestimmen.

3. Einrichtung (42) nach einem der Ansprüche 1 oder 2, wobei die Verarbeitungseinheit (46) weiter dazu konfiguriert ist, ein Muster von Rändern und/oder Leisten von dermalen Papillen aus dem einen oder den mehreren Bildern zu bestimmen.

4. Einrichtung (42) nach einem der Ansprüche 1- 3, wobei die Verarbeitungseinheit (46) dazu konfiguriert ist, ein trainiertes maschinelles Lernmodell zu verwenden, um das eine oder die mehreren Bilder zu verarbeiten, um den Hauttyp und/oder den Melaninindex der Haut des Subjekts auf Basis der Dichte der Struktur des Pigmentnetzes zu bestimmen.

5. Einrichtung (42) nach einem der Ansprüche 1-4, wobei der bestimmte Hauttyp einen umfasst von: normaler Haut, trockener Haut, fettiger Haut, gebräunter Haut, ungebräunter Haut, Mischhaut und einem Hauttyp auf der Fitzpatrick-Skala.

6. Einrichtung (42) nach einem der Ansprüche 1-5, wobei die Einrichtung (42) weiter eine Benutzerschnittstelle umfasst, die dazu konfiguriert ist, das erste Signal zu empfangen, und wobei das erste Signal dazu konfiguriert ist, zu bewirken, dass die Benutzerschnittstelle Rückmeldung, die den bestimmten Hauttyp und/oder Melaninindex angibt, für einen Benutzer ausgibt.

7. Einrichtung (42) nach einem der Ansprüche 1-6, wobei das erste Signal an eine Steuereinheit einer Behandlungsvorrichtung (2) ausgegeben wird, und wobei die Steuereinheit das erste Signal verwendet, um eine oder mehrere Betriebseinstellungen für die Behandlungsvorrichtung (2) zu bestimmen.

8. Einrichtung (42) nach einem der Ansprüche 1-7, wobei die Verarbeitungseinheit (46) dazu konfiguriert ist, das eine oder die mehreren empfangenen Bilder hinsichtlich Farbe zu normalisieren und das eine oder die mehreren normalisierten Bilder zu verarbeiten, um den Hauttyp und/oder Melaninindex der Haut zu bestimmen.

9. System, umfassend:
eine Bildgebungseinheit (44), die dazu eingerichtet ist, Bilder der Haut eines Subjekts zu erhalten; und
eine Einrichtung (42) nach einem der Ansprüche 1-8.

10. System nach Anspruch 9, weiter umfassend:
eine oder mehrere Lichtquellen (12) zum Beleuchten der Haut.

11. System nach Anspruch 10, wobei die eine oder die mehreren Lichtquellen (12) dazu konfiguriert sind, Licht zu emittieren, das Wellenlängen im sichtbaren Spektrum aufweist, und wobei das emittierte blaue Licht eine höhere Intensität aufweist als Licht anderer Farben.

12. Computerimplementiertes Verfahren zum Bewerten der Haut eines Subjekts, wobei das Verfahren umfasst:
Empfangen (101) eines oder mehrerer Bilder der Haut des Subjekts von einer Bildgebungseinheit, wobei die Bildgebungseinheit dazu eingerichtet ist, Bilder der Haut des Subjekts zu erhalten;
Verarbeiten (103) des einen oder der mehreren Bilder, um einen Hauttyp und/oder einen Melaninindex der Haut des Subjekts zu bestimmen;
Ausgeben (105) eines ersten Signals, das den bestimmten Hauttyp und/oder Melaninindex angibt;
**dadurch gekennzeichnet, dass** die Bestimmung des Hauttyps und/oder des Melaninindex der Haut auf einer Dichte einer Struktur eines Pigmentnetzes der Haut basiert.

13. Computerprogrammprodukt, das ein computerlesbares Medium umfasst, das darin verkörperten computerlesbaren Code aufweist, wobei der computerlesbare Code derart konfiguriert ist, dass bei Ausführung durch einen geeigneten Computer oder eine geeignete Verarbeitungseinheit der Computer oder die Verarbeitungseinheit veranlasst wird, das Verfahren nach Anspruch 12 durchzuführen.

## Revendications

1. Appareil (42) pour évaluer la peau d'un sujet, l'appareil (42) comprenant une unité de traitement (46) configurée pour :
recevoir une ou plusieurs images de la peau du sujet en provenance d'une unité d'imagerie (44), dans lequel l'unité d'imagerie (44) est agencée pour obtenir des images de la peau du sujet ;
traiter les une ou plusieurs images pour déterminer un type de peau et/ou un indice de mélanine de la peau du sujet ; et
émettre un premier signal indiquant le type de peau déterminé et/ou l'indice de mélanine déterminé ;
**caractérisé en ce que** la détermination du type de peau et/ou de l'indice de mélanine de la peau est basée sur une densité d'une structure d'un réseau pigmentaire de la peau.

2. Appareil (42) selon la revendication 1, dans lequel l'unité de traitement (46) est en outre configurée pour déterminer une densité de mélanine dans des kératinocytes et/ou des mélanocytes dans la peau à partir des une ou plusieurs images.

3. Appareil (42) selon l'une quelconque des revendications 1 ou 2, dans lequel l'unité de traitement (46) est en outre configurée pour déterminer un motif de brides et/ou de crêtes de papilles dermiques à partir des une ou plusieurs images.

4. Appareil (42) selon l'une quelconque des revendications 1-3, dans lequel l'unité de traitement (46) est configurée pour utiliser un modèle d'apprentissage automatique entraîné pour traiter les une ou plusieurs images pour déterminer le type de peau et/ou l'indice de mélanine de la peau du sujet sur la base de la densité de la structure du réseau pigmentaire.

5. Appareil (42) selon l'une quelconque des revendications 1-4, dans lequel le type de peau déterminé comprend l'un quelconque : d'une peau normale, d'une peau sèche, d'une peau grasse, d'une peau bronzée, d'une peau non bronzée, d'une peau mixte et d'un type de peau sur l'échelle de Fitzpatrick.

6. Appareil (42) selon l'une quelconque des revendications 1-5, dans lequel l'appareil (42) comprend en outre une interface utilisateur configurée pour recevoir le premier signal et dans lequel le premier signal est configuré pour amener l'interface utilisateur à délivrer un retour d'information à un utilisateur indiquant le type de peau déterminé et/ou l'indice de mélanine déterminé.

7. Appareil (42) selon l'une quelconque des revendications 1-6, dans lequel le premier signal est émis à une unité de commande d'un dispositif de traitement (2) et dans lequel ladite unité de commande utilise le premier signal pour déterminer un ou plusieurs paramètres fonctionnels pour le dispositif de traitement (2).

8. Appareil (42) selon l'une quelconque des revendications 1-7, dans lequel l'unité de traitement (46) est configurée pour normaliser les une ou plusieurs images reçues pour une couleur, et pour traiter les une ou plusieurs images normalisées pour déterminer le type de peau et/ou l'indice de mélanine de la peau.

9. Système, comprenant :
une unité d'imagerie (44) agencée pour obtenir des images de la peau d'un sujet ; et
un appareil (42) selon l'une quelconque des revendications 1-8.

10. Système selon la revendication 9, comprenant en outre :
une ou plusieurs sources de lumière (12) pour éclairer la peau.

11. Système selon la revendication 10, dans lequel les une ou plusieurs sources de lumière (12) sont configurées pour émettre de la lumière présentant des longueurs d'onde dans le spectre visible et dans lequel une lumière bleue émise présente une intensité plus élevée qu'une lumière d'autres couleurs.

12. Procédé mis en œuvre par ordinateur pour évaluer la peau d'un sujet, le procédé comprenant :
la réception (101) d'une ou de plusieurs images de la peau du sujet en provenance une unité d'imagerie, l'unité d'imagerie étant agencée pour obtenir des images de la peau du sujet ;
le traitement (103) des une ou plusieurs images pour déterminer un type de peau et/ou un indice de mélanine de la peau du sujet ;
l'émission (105) d'un premier signal indiquant le type de peau déterminé et/ou l'indice de mélanine déterminé,
**caractérisé en ce que** la détermination du type de peau et/ou de l'indice de mélanine de la peau est basée sur une densité d'une structure d'un réseau pigmentaire de la peau.

13. Produit de programme informatique comprenant un support lisible par ordinateur dans lequel est incorporé un code lisible par ordinateur, le code lisible par ordinateur étant configuré de telle sorte que, lors de son exécution par un ordinateur approprié ou une unité de traitement appropriée, l'ordinateur soit amené, ou l'unité de traitement soit amenée, à réaliser le procédé selon la revendication 12.
